## Europäisches Patentamt

## European Patent Office

## Office européen des brevets

(11) Publication number: **0 216 832**
**B1**

# EUROPEAN PATENT SPECIFICATION

(45) Date of publication of patent specification: **29.08.90**

(51) Int. Cl.⁵: **C 07 K 7/40, A 61 K 37/26 // C12P21/02**

(21) Application number: **86901826.7**

(22) Date of filing: **14.03.86**

(86) International application number:
**PCT/DK86/00022**

(87) International publication number:
**WO 86/05496 25.09.86 Gazette 86/21**

(54) **NOVEL INSULIN DERIVATIVES AND PHARMACEUTICAL PREPARATIONS CONTAINING THESE DERIVATIVES.**

(30) Priority: **15.03.85 DK 1197/85**

(43) Date of publication of application:
**08.04.87 Bulletin 87/15**

(45) Publication of the grant of the patent:
**29.08.90 Bulletin 90/35**

(84) Designated Contracting States:
**AT BE CH DE FR GB IT LI LU NL SE**

(56) References cited:
**EP-A-0 045 187**
**EP-A-01 948 64**
**DK-B-14 648 2**
**DK-B-14 743 7**
**US-A-3 903 068**
**US-A-44 216 85**

**Biochimica et Biophysica Acta, 33 (1973), p 406-415 D Levy, The Esterification of Insulin with Triethyloxonium Tetrafluoroborat. Chemical Abstracts, Vol. 87 (1977), abstract No. 161917r Hoppe- Seyler's Z. Physiol. Chem. 1977, 358 (1), p 105-113 (Eng.)**

(73) Proprietor: **NOVO-NORDISK A/S**
**Novo Allé**
**DK-2880 Bagsvaerd (DK)**

(72) Inventor: **BALSCHMIDT, Per**
**Tibberup Alle 20**
**DK-3060 Espergaerde (DK)**
Inventor: **HANSEN, Finn, Benned**
**Oesterbrogade 54 B**
**DK-2100 Copenhagen (DK)**

(74) Representative: **Christensen, Aksel et al**
**c/o Dansk Patent Kontor A/S H.C. Orsteds Vej 70, Postbox 237**
**DK-1879 Frederiksberg C (DK)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to novel insulin derivatives and to insulin preparations showing protracted action and comprising at least one of the novel insulin derivatives and, if desired, a fast acting insulin.

In severe or chronic cases the disease of Diabetes is usually treated with injection preparations containing insulin, e.g. porcine insulin, bovine insulin or human insulin.

Soluble insulin preparations are usually fast acting, but in return the action ceases after few hours. Therefore, it is necessary to give frequent injections, normally several times a day.

In order to overcome this disadvantage insulin preparations with protracted action have been formulated so that the action is maintained for several hours or even up to 24 hours or longer. Using such protracted preparations the diabetic patient only has to receive a smaller number of injections, e.g. a single or two injections during 24 hours.

Such a protracted action can be achieved by converting the insulin to a slightly soluble salt, such as zinc insulin or protamin insulin. The slightly soluble insulin salts are used in the form of suspensions from which the insulin is gradually released after subcutaneous injection.

Recently other methods have been invoked to achieve a protracted action. An example hereof is the encapsulation of insulin crystals is polymerized serum albumin. Another example is continuously acting infusion devices, so-called insulin pumps, which however may be uncomfortable and entail a risk to the patient.

DK—A—3582/84 3583/84 disclose the preparation and use of insulin derivatives wherein the C-terminus of the B-chain is extended with an organic group carrying at least one positive charge, preferably Arg-OH or Arg-Arg-OH. Preparations containing suspensions of such insulin derivatives exhibit a protracted action.

According to US—A—4 343 898 a human insulin-Thr(B30) ester, in which the ester residue is a lower alkyl group up to 4 C atoms or an aralkyl group, can be obtained from, per example, crude porcine insulin.

EP—A—0 045 187 describes that human insulin can also be prepared from threonine and porcine insulin by ancymatic of the B30 amino acid to give inter alia insulin (B30) alkyl esters or alkyl amides, the alkyl group of which are straight or branched and contain from 1 to 6 C atoms.

In EP—A—0 194 864 new insulin derivatives having a positive charge which can be used to prepare solutions having prolonged insulin action are described. In these derivatives the B30 carboxyl group has been blocked with an amido residue having an alkyl group with less than 7 C atoms or by an ester residue containing up to 4 C atoms, the B27 amino acid has been substituted by a basic amino acid and/or a neutral amino acid has been inserted in the A4- A17-, A13- and/or B21-position.

Hoppe Seyler's Z. Phyisiol. Chem. Vol. 358, p. 105—113 (1977) discloses the saponification of insulin hexamethyl ester yielding A21-asparaginimide-insulin with decreased biological activity.

Biochem. Biophys. Acta, Vol. 310, p. 406—415 (1973) refers to the esterification of insulin to the B30-alaninethylester.

None of these insulin derivatives or preparations is satisfactory with respect of a long-lasting protecting action.

It has now surprisingly be found that by the use of insulin derivatives, wherein the B30-carboxyl group is blocked with an uncharged group and optionally 1 to 4 of the carboxylic acid groups on the amino acid residues of positions A4, A17, A21, B13 and B21 are blocked with or converted into an uncharged group, in insulin preparations, in particular injection preparations, a hitherto unknown and desirably long-lasting protectic action is achieved. This effect is particularly pronounced, if the B30 carboxyl group is blocked with a group containing an alkyl chain of at least 8 carbon atoms, or if the B30-carboxyl group is blocked with an uncharged group and at least one and not more than four additional carboxyl groups at the same time are blocked with or converted into an uncharged group.

It is therefore the object of the invention to provide novel insulin derivatives and preparations containing them having a long-lasting protracted insulin action.

This object is achieved according to claims 1, 4 and 9. The dependent claims concern preferred embodiments.

Preferred insulin derivatives are those wherein only the B30 carboxyl group is blocked with an uncharged group which comprises an aliphatic alkyl group of 8—12 carbon atoms, the B30 carboxyl group is blocked as an alkyl amide having at least 8 carbon atoms, one of the carboxylic acid groups on the amino acid residues of positions A4, A17, B13 and B21 is blocked with or converted into an uncharged group and the B30 carboxyl group is blocked with an uncharged group comprising an aliphatic alkyl group of 8—12 carbon atoms.

The invention also relates to insulin preparations containing at least one insulin derivative wherein the B30 carboxyl group is blocked with an uncharged group and optionally one to four of the carboxylic acid groups on the amino acid residues of positions A4, A17, A21, B13 and B21 are blocked with or converted into an uncharged group, provided that when only the B30 carboxyl group is blocked with an uncharged group, said group comprises an alkyl group having at least 8 carbon atoms and, if desired, also containing a fast acting insulin.

A particularly advantageous embodiment of the insulin preparations of the invention are injection preparation exhibiting protracted insulin action and comprising a suspension of at least one insulin derivative of the invention in an isotonic aqueous medium having a neutral pH and optionally containing a buffer and/or a preserving agent and, if desired, a fast acting insulin.

Insulins which are normally used for the treatment of Diabetes, such as porcine, bovine, ovine, and human insulin, do all contain six free carboxylic groups, viz. in the A4-, A17-, A21-, B13-, B21- and B30-amino acid residues. The numbering is referring to the positions in the insulin A and B chain, respectively, starting from the N termini. It is known that an insulin in which the B30 carboxyl group is converted into a methyl ester or amide possesses unchanged biological activity, vide H. Zahn, et al.: Naturwissenschaften, 68, (1981), p. 56—62, and M. Kobayashi, et al.: Diabetes, 30 (1981), p. 519-22. A protracted action was not mentioned. The biological activity of insulin will usually decrease by an increasing degree of derivatization. However, the biological activity is influenced surprisingly little even at high degrees of derivatization of the free carboxylic acid groups; however, when all six free carboxylic acid groups are derivatized the biological activity is completely abolished, vide D. Levy: Biochem. Biophys. Acta, 310 (1973), pages 406—415.

By means of the insulin preparations of the invention the insulin activity can be maintained, and at the same time a surprisingly protracted action can be achieved. This protracting effect is dependent on the properties of the specific derivative, i.e. not only on the number of carboxylic acid groups being blocked, but also on the size and the chemical composition of the blocking group. Thus, it is possible to vary the protracted action at will.

In the case of Insulin-Dependent Diabetes a frequently used therapy consists in two daily injections of a protracted insulin preparation, one in the morning and one in the evening just before bedtime, in order to create a basic insulin level. Additionally, three injections of a fast acting insulin preparation are given prior to the principal meals. The disadvantage of this therapy is that the late injection of the protracted preparation may result in a dangerously low blood glucose level in the course of the night. This situation may be avoided by injecting a mixed preparation of a protracted and a fast acting insulin before supper, whereby hypoglycemia will, if at all, occur during the evening, where it can be averted by means of a light snack. However, this type of the therapy often results in hyperglycemia during the morning, as the most used protracted insulin preparations "Insulatard"® and "Monotard"® do not act long enough. Therefore, it is desirable to provide the diabetic patient with insulin preparations that act longer than the preparations commonly in use, in particular if one injection of such preparations will suffice for one or even several days. Insulin preparations prepared according to the invention exhibit a protracted insulin action of the same or longer duration than that of the commonly used protracted insulin preparation "Insulatard"®.

With particular insulin derivatives of the invention in which only the B30 carboxyl group is blocked with an uncharged group, derivatives are preferred in which the blocking takes place in the form of alkyl ester or alkyl amide, wherein the alkyl group contains from 8 to 18 carbon atoms, in particular in the form of N-alkyl. If the alkyl group is smaller, the prolongation will not become substantially different from that of "Insulatard"®, and if the alkyl group is larger, solubility problems will arise which make the preparation of the derivatives very difficult.

Among the insulin derivatives of the invention wherein the B30 carboxyl group and additionally from 1 to 4 carboxyl groups are blocked with an uncharged group, derivatives are preferred in which the blocking takes place in the form of amide, alkyl ester and, as far as the B30 carboxyl group is concerned, also in the form of alkyl amide, whereby an alkyl group in the B30 carboxyl group may contain up to 18 carbon atoms, but in the remaining carboxyl groups not more than four carbon atoms only.

The invention relates in particular to the following specific compounds:

Insulin-B30-octyl ester, insulin-B30-dodecyl amide, insulin-B30-hexadecyl amide, insulin-(B21,B30)-dimethyl ester, insulin-(A17,B30)-dimethyl ester, insulin-(A4,B30)-diamide, insulin-A17-amide-B30-octyl ester, insulin-(A4,B13)-diamide-B30-hexyl amide, insulin-(A4,A17,B21,B30)-tetramide, insulin-(A17,B30)-diamide, A4-ala-insulin-B30-amide, B30-leu-insulin-(A4,B30)-diamide.

When the insulin preparations of the invention contain a fast acting insulin, this insulin may e.g. be human or porcine insulin.

The insulin derivatives in question may be derived from porcine, bovine, ovine, rabbit, or in particular human insulin, including other such insulins wherein the B30 amino acid residue has been exchanged with another naturally occurring amino acid residue, e.g. by semisynthesis.

The insulin derivatives can be prepared in a manner known per se for the conversion of a carboxylic acid group into an uncharged group, e.g. by esterification or amidation. Thus, an insulin derivative wherein two or more of the total of six carboxylic acid groups are methylated, is e.g. prepared by reacting human insulin-B30-methyl ester in borontrifluoride/methanol for a suitable time, followed by a fractionation of the modified insulin derivatives on an anion exchange column at a slightly basic pH value. Finally, the possible products can be fractionated and isolated by preparative HPLC.

Derivatization of the B30 carboxyl group can take place by enzymatic catalysis, e.g. by following the process disclosed in DK—A—146,482. According to this process the B30-amino acid is removed by digesting e.g. porcine insulin with carboxypeptidase A, and in the second step the des-B30-insulin formed is reacted in the presence of trypsin or a trypsin-like enzyme with a neutral amino acid ester, especially the threonine methyl ester.

A similar process is known from DK—A—No. 574/80, according to which process the B30-amino acid in an insulin by a one pot method is exchanged by tryptic catalysis with the desired amino acid ester or amide.

According to the above application the reaction may even be carried out using as starting material a proinsulin.

Some the carboxyl group blockings preferred according to the invention can be established by biotechnological methods. This applies to the blocking of the carboxyl groups in the positions of A4, A17, B13 and B21 in the form of amides, or by changing some of or all the glutamic acids located at these positions with naturally occurring amino acids carrying an uncharged side chain.

Usually the amino acids in these positions are glutamic acid, but in some of the insulin derivatives of the invention they are exchanged with glutamine.

It is obvious to prepare these modified insulins by biosynthesis, as by changing only a single base in the codon in the DNA strand coding for glutamic acid this codon can be made code for e.g. glutamine. By changing bases in the DNA strand coding for insulin (or proinsulin) it is thus possible to make modifications which would be difficult or impossible to make chemically due to limitations as to isolation.

There exists a number of different methods to prepare human insulin by biosynthesis. It is common to all of them that the DNA strand coding for either the entire proinsulin, a modified version thereof or the A- and B-chain separately has been inserted into a replicable plasmide containing a suitable promoter. By transforming this system into a given host organism a product can be produced, which can be converted into authentic human insulin in a manner known per se.

Some known methods for biosynthesis of proinsulin or A- and B-chain and their conversion to insulin are described below.

In the well known Genentech process an intracellular preparation in E. coli of polypeptides is made, wherein these polypeptides are a fusion between β-galactosidase and the A- and B-chain respectively, of insulin. After fermentation the two products are isolated, the A- and B-chains are split off from β-galactosidase by means of cyanogen bromide, whereafter the sulphitated chains are combined under reducing conditions in the presence of dithiothreitol at a pH value of 10.5. Finally human insulin is isolated from this reaction mixture.

In the process disclosed in EP—A—55945 the product is synthetized as a fusion protein with another protein separated by a cleaving site. This may be a protease cleaving site or a site which can be cleaved chemically (e.g. methionine). The gene for proinsulin is e.g. cloned as a gene fusion with another protein by means of recombinant DNA techniques. After isolation, the chimeric protein is cleaved by using a suitable enzyme or CNBr, whereupon the denatured proinsulin is isolated in a sulphitolyzed form. This product is renatured under reducing conditions using β-mercapto ethanol at a pH of 10.5, as described by Frank, followed by trypsin/CpB cleavage as described by Kemmler (1971). The resulting insulin is isolated in a manner known per se.

Finally, proinsulin can be prepared biosynthetically by using the method disclosed in EP—A—116201. In this method proteins are secreted into the culture medium by using the α-factor system from Saccharomyces cerevisiae. By inserting the gene coding for proinsulin into this system, proinsulin can be isolated from the culture medium by using the system described in DK patent application No. 3091/84. Hereafter, proinsulin can be converted into insulin by the known methods described above.

In the above, methods are described in which the product is either proinsulin or A- and B-chain separately. Furthermore, these products are either synthetized together with a signal sequence, the purpose of which is to bring the product through to the cell surface where it is split, or as a fusion with a protein, the purpose of which is to stabilize the product.

Moreover it is possible to prepare modified proinsulins biosynthetically. EP—A—82303071 discloses proinsulins wherein the C-chain is modified by means of recombinant DNA techniques.

The above-mentioned modifications of insulin can be prepared by expressing the modified DNA sequence in a suitable expression system. The changes of the DNA sequence can be made by *in vitro* mutagenesis of the insulin gene by a method described by Thomas A. Kunkel, Proc. Natl. Acad. Sci., USA, Vol 82, pp. 448—492 (1985). According to this method the insulin gene is cloned into the single-stranded DNA bacteriophage M13. DNA from this phage is purified, and hereto a primer, typically a 15-25-mer, which contains the given mutation (one mismatch) as well as a homology on each side of this mutation is annealed. The primer is then extended by use of a DNA polymerase in the total length of the phage genome and thus provides the complete double-strand molecule, wherein one strand contains the mutation, and the other contains the wild type gene. By introducing this molecule into an E. coli cell, the phage progeny will partly be phages containing the wild type gene, partly phages containing the mutation. Among these phages it is then possible to screen for the desired type by hybridization with the mutagenization primer to single-stranded DNA of the phage progeny. The primer will have complete homology to the mutated strand, but will be different from the wild type at a single nucleotide. After screening, double strand DNA can be isolated from the E. coli cells, wherein the phage is replicated, and the modified insulin gene can be isolated herefrom. This gene is thereafter reinserted into the original expression system.

The injection preparations can comprise the insulin derivatives of the invention as amorphous and/or crystalline suspensions, either separately or in combination with other such insulin derivatives and/or naturally occurring insulins. It has been found that a majority of these derivatives can be crystallized with zinc, either at a pH value being higher than their isoelectric points, or at a pH value of about the isoelectric pH value, when the crystallization medium contains a phenol. It is also possible to crystallize the insulin derivatives of the invention in the presence of protamin or an excess of zinc, which causes a further protracted activity.

The invention is further illustrated by means of the following examples. However, Example 5 illustrates the preparation of an insulin derivative falling outside the scope of the invention and used as comparative compound in Example 6. Example 9 illustrates the preparation of starting materials used in Examples 1 and 5.

Example 1
Preparation of human insulin-B30-n-dodecyl amide

970 mg of L-threonine-n-dodecyl amide were dissolved in a mixture of 3.60 ml of t.-butanol, 1.20 ml of dimethyl formamide and 1.40 ml of water. 450 mg of des-B30-alanine porcine insulin were dissolved in the mixture with stirring and the pH value adjusted to 6.4—6.5 with acetic acid. A solution of 8 mg of trypsin in 0.20 ml of 0.001 M calcium acetate was added and the reaction mixture left at 20°C. for 2 hours. A sample was analyzed by reverse phase high pressure liquid chromatography which showed that 71% of the des-B30-insulin was converted into insulin-B30-n-dodecyl amide.

The reaction mixture was poured into 5 ml of 2-propanol and left for 15 minutes. The precipitated protein was isolated by centrifugation and the precipitate washed with 25 ml of 2-propanol and centrifuged again.

The precipitate was redissolved in 15 ml of 1 M acetic acid in 7 M urea, and after filtration the solution was applied to a 2.6×90 cm column packed with Sephadex® G50 Superfine and equilibrated with 1 M acetic acid. The protein was eluted with the same buffer at a rate of 25 ml per hour, and fractions of 5 ml were collected. The optical density at 277 nm of the fractions were examined, whereupon the proteinaceous main fraction was collected and freeze-dried.

The freeze-dried protein was redissolved in 25 ml of 60% by volume ethanol and the pH adjusted to 9.0 with 1 M TRIS. After filtration, the solution was applied to a 1.6×20 cm column of Q-Sepharose® CL-6B FF, equilibrated with 0.02 M TRIS/hydrochloric acid in 60% by volume ethanol, pH 8.25.

The column was eluted at 20°C. at a rate of 50 ml per hour, first for 1.5 hour with equilibration buffer and then with a linear gradient of from 0 to 0.1 M sodium chloride in the same buffer for 16 hours. The proteinaceous main fraction eluted at a salt strength of about 0.05 M sodium chloride was collected, whereupon the ethanol was removed by evacuation. The pH value of the aqueous residue was adjusted to 6.3 with hydrochloric acid and centrifuged after standing at 4°C. for 1 hour, whereafter the isolated human insulin-B30-n-dodecyl amide was freeze-dried. Yield: 96 mg.

Example 2
Formulation and biological effect of an insulin preparation containing human insulin-B30-n-dodecyl amide.

30 mg of human insulin-B30-n-dodecyl amide were dissolved in 10 ml of 0.05 M m-cresol by the addition of a small amount of hydrochloric acid, whereafter the solution was sterilized by filtration. 5 ml of a sterile solution being 0.05 M in respect to sodium dihydrogen phosphate and 0.7 M in respect to glycerol were then added to the solution, whereupon the pH value was adjusted to 7.3 with sodium hydroxide solution. Finally, the volume was increased to 20 ml by addition of sterile water.

When the resulting suspension preparation was injected subcutaneously into guinea pigs (20 µ litres per kg) hypoglycemia persisted substantially longer than the hypoglycemic effect produced by the standard NPH insulin preparation "Insulatard"®.

Example 3
Preparation of human insulin-(B21,B30)-dimethyl ester and human insulin-(A17,B30)-dimethyl ester, respectively.

500 mg of human insulin-B30-methyl ester were suspended in 50 ml of dry methanol. 2.0 ml of 20% by weight borontrifluoride in methanol were added with stirring. After standing at ambient temperature for 2 hours the sample was poured into 400 ml of ether and centrifuged for 20 minutes at 2500×g, 4°C. The precipitate was washed twice with ether, resuspended in 150 ml of ether, evaporated in vacuo and dissolved in 50 ml of 20 mM TRIS/hydrochloride, 60% by volume ethanol, pH value 8.25. The sample was then applied to a 1.6×21 cm Q-Sepharose® CL-6B Fast Flow column equilibrated with the same buffer. After 1 hour of isocratic elution at a flow rate of 24 cm/hour the sodium chloride concentration was increased linearly from 0 to 0.1 M during the following 16 hours. The peak containing insulin dimethyl ester was collected in 3 main pools.

The first eluted pool was further fractionated on a 4×250 mm LiChrosorb® RP18 column (Merck 50333) equilibrated with a buffer containing 0.125 M ammonium sulphate, pH value 4.0 and acetonitrile. 1—2 mg of protein was applied to the column and eluted with the above buffer containing 38% acetonitrile. The main proteinaceous fraction eluted after 20—25 minutes. The acetonitrile was removed from the sample in vacuo and the protein applied to a SepPak® C18 column (Waters Ass. No. 51910), washed with $H_2O$ and eluted with 70% methanol. The latter was removed in vacuo, and the protein was freeze-dried. Hereby 20 ml of human insulin-B21,B30-dimethyl ester were obtained.

The next eluted pool was treated in a corresponding manner, whereby 15 mg of human insulin-A17,B30-dimethyl ester were obtained.

5

Both human insulin dimethyl esters were characterized by Plasma Desorption Mass Spectrometry, whereby the correct molecular weight was found, and by decomposition with S. Aureus protease, whereby the esterified glutamic acids were identified.

Example 4

Bological effect of preparations containing human insulin dimethyl esters

25 ml of a medium containing 1.6% (w/v) of glycerol, 0.33% (w/v) of m-cresol, 1/75 M of sodium phosphate, pH value 7.3, were prepared. The solution was sterilized by filtration. 2.1 mg of human insulin-(B21,B30)-dimethyl ester, prepared as described in Example 3, were suspended in 1.0 ml of medium.

Analogously, 3.2 mg of human insulin-(A17,B30)-dimethyl ester, prepared as described in Example 3, were suspended in 1.0 ml of medium.

Strongly protracted hypoglycemia was observed by administering these zinc-free preparations subcutaneously to guinea pigs. As it is apparent from the Table below, the protraction is comparable to the commonly used crystalline protamin-insulin preparation "Insulatard"®.

| Time (hours) | 0 | 1 | 2 | 4 | 6 |
|---|---|---|---|---|---|
| Insulatard[R] 11 µg/kg | 100 | 74 | 45 | 82 | 98 |
| Human insulin-(B21,B30)-dimethyl ester 16 µg/kg | 100 | 64 | 51 | 79 | 94 |
| Human insulin-(A17,B30)-dimethyl ester 24 µg/kg | 100 | 50 | 38 | 55 | 94 |

The Table shows the blood glucose value in % of the starting value (n=6 animals).

Example 5

Preparation of human insulin-B30-n-hexyl amide

290 mg of L-threonine-n-hexyl amide, prepared as described in Example 9, were dissolved in a mixture of 2.60 ml of 96% by volume ethanol, 0.80 ml of water and 0.16 ml of acetic acid. 240 mg of des-B30-alanine porcine insulin were added with stirring and the pH value adjusted to 6.3—6.4 with triethylamine. A solution of 5 mg of trypsin in 0.10 ml of 0.001 M calcium acetate was then added and the reaction mixture left at 12°C. After two hours the reaction mixture was poured into 20 ml of 2-propanol, and after standing for 15 minutes the precipitated protein was isolated by centrifugation. The precipitate was washed with 20 ml of 2-propanol and centrifuged again.

Analysis of the precipitate by reverse phase high pressure liquid chromatography showed a conversion of 65%.

The precipitate was redissolved in 10 ml of 1 M acetic acid in 7 M urea, and, after filtration, the solution was applied to a 2.6×90 cm Sephadex® G50 Superfine column equilibrated with 1 M acetic acid. This column was eluted with the same buffer at a rate of 25 ml per hour. The proteinaceous main fraction was collected and freeze-dried.

The freeze-dried protein was redissolved in 20 ml of 60% by volume ethanol by adjusting the pH value to 8.25 with 1 M tris-(hydroxymethyl)-amino methane (TRIS). After filtration, the solution was applied to a 1.5×20 cm Q-Sepharose® CL-6B FF column, equilibrated with 0.02 M TRIS/-hydrochloric acid in 60% by volume ethanol, pH 8.25. The column was eluted at 20°C. at a rate of 50 ml per hour, first with equilibration buffer for 1.5 hour and for the following 16 hours with a linear gradient of from 0 to 0.1 M sodium chloride. The protein material eluting at a salt strength of ~0.05 M sodium chloride was collected, whereupon the ethanol was removed in vacuo. The pH value of the aqueous evaporation residue was adjusted to 6.3 with hydrochloric acid and centrifuged after standing at 4°C. for 4 hours. The isolated human insulin-B30-n-hexyl amide was freeze-dried. Yield: 50 mg.

Example 6

Formulation and biological effect of an insulin preparation containing human insulin-B30-n-hexyl amide

30 mg of human, insulin-B30-n-hexyl amide were dissolved in 10 ml of 0.05 M m-cresol by adding a small amount of hydrochloric acid, whereupon the solution was sterilized by filtration. 5 ml of a sterile solution being 0.05 M in respect to sodium dihydrogen phosphate and 0.7 M in respect to glycerol were added to the solution and the pH value adjusted to 7.3 with a sterile sodium hydroxide solution. Finally, sterile water was added to give a final volume of 20 ml.

Subcutaneous injection (20 µ litres/kg) of this preparation into guinea pigs resulted in a protracted hypoglycemia corresponding to the hypoglycemia induced by a standard NPH insulin preparation ("Insulatard"®).

Example 7
Preparation of human insulin-A4-amide-B30-ethyl ester

50 mg of human proinsulin, wherein the glutamic acid of position A4 has been replaced by glutamine, introduced by one of the biotechnological methods described in the introductory part, were dissolved in a mixture containing 400 µ litres of N,N-dimethyl formamide, 100 µ litres of water, and 125 mg of L-threonine ethyl ester. The pH value of the solution was adjusted to 6.5 with acetic acid. 2.5 mg of porcine trypsin, dissolved in 100 µ litres of 0.001 M calcium acetate, was added and the reaction mixture left at 12°C. The reaction was stopped after 48 hours by precipiting the proteins with 10 ml of 2-propanol. The precipitate was isolated by centrifugation and subsequently fractionated by gelfiltration and ion-exchange chromatography as described in Example 5. Hereby 15 mg of human insulin-A4-amide-B30-ethyl ester were obtained.

Example 8
Formulation and biological effect of an insulin preparation containing human insulin-A4-amide-B30-ethyl ester

15 mg of human insulin-A4-amide-B30-ethyl ester were suspended in 5 ml of an aqueous solution containing 15 mg of sodium acetate trihydrate, 400 mg of mannitol, and 30 mg of phenol, whereupon the pH value was adjusted to 7.3, and water was added to give a final volume of 10 ml.

Subcutaneous injection (20 µ litres/kg) of this preparation into guinea pigs resulted in a hypoglycemia of longer duration than the hypoglycemia induced by a standard NPH-insulin preparation ("Insulatard"®).

Example 9
Preparation of L-threonine alkyl amides

10 mmoles of t-butyloxycarbonyl-L-threonine and 10 mmoles of alkylamine were dissolved in 25 ml of chloroform, whereupon 11 mmoles of N-ethyloxycarbonyl-2-ethyloxy-1,2-dihydroquinoline (EEDQ) were added. The reaction mixture was stirred at 20°C. for 16 hours, whereupon the solvent was removed by evaporation in vacuo. The resulting viscous oil was redissolved in 100 ml of ethyl acetate, washed with 2×50 ml of 10% citric acid, 2×50 ml of 1 M sodium bicarbonate and 2×50 ml of water. The organic phase was dried over anhydrous sodium sulphate and removed by evaporation in vacuo.

To splitt off the protecting group the residue was dissolved in 25 ml of trifluoroacetic acid and left at 20°C. for 30 minutes. After removal of trifluoroacetic acid by evaporation the resulting viscous oil was mixed with 50 ml of 1 M sodium carbonate and the mixture extracted with 3×50 ml of chloroform. The combined extracts were dried over anhydrous sodium sulphate and evaporated in vacuo. The residue solidified to waxy crystals upon standing at 4°C. Yield: 50—85%.

**Claims**

1. Insulin derivatives, wherein the B30 carboxyl group is blocked with an uncharged group, characterized in that the B30 carboxyl group of the insulin molecule is blocked with an uncharged group comprising an aliphatic alkyl group having at least 8 carbon atoms.

2. Insulin derivatives according to claim 1, characterized in that the B30 carboxyl group is blocked with an uncharged group comprising an aliphatic alkyl group of 8 to 12 carbon atoms.

3. Insulin derivatives according to claim 1, characterized in that the B30 carboxyl group is blocked as an alkyl amide having at least 8 carbon atoms.

4. Insulin derivatives, wherein the B30 carboxyl group and one to four of the carboxylic acid groups on the amino acid residues of positions A4, A17, B13 and B21 of the insulin molecule are blocked with or converted into uncharged groups, characterized in that the B30 carboxyl group is blocked as an amide or with an uncharged group comprising an aliphatic alkyl group of 1 to 12 carbon atoms, whereby Gln (A17), Gln (B13), Thr (B30)-NH$_2$ human insulin, Gln (A17), Thr (B30)-NH$_2$ human insulin and Gln (B13), Thr (B30)-NH$_2$ human insulin are excluded.

5. Insulin derivatives according to claim 4, characterized in that the B30 carboxyl group is blocked with an uncharged group comprising an aliphatic alkyl group of 8 to 12 carbon atoms.

6. Insulin derivative according to claim 4, characterized in that it is an insulin-(A4, B30) diamide.

7. Insulin derivatives according to any one of claims 1 to 6, characterized in that they are human insulin derivatives.

8. Insulin derivatives according to any one of claims 1 to 6, characterized in that they are porcine derivatives.

9. Insulin preparations, characterized in that they contain at least one insulin derivative according to any one of claims 1 to 8.

10. Insulin preparations according to claim 9, characterized in that they contain also a fast acting insulin.

11. Preparations according to claim 9 or 10, characterized in that they are injection preparations exhibiting protracted insulin action and comprise a suspension of at least one insulin derivative in an isotonic aqueous medium having a neutral pH and optionally contain a buffer and/or a preserving agent.

# EP 0 216 832 B1

**Patentansprüche**

1. Insulinderivate, bei denen die B30-Carboxylgruppe mit einer ungeladenen Gruppe blockiert ist, dadurch gekennzeichnet, daß die B30-Carboxylgruppe des Insulinmoleküls mit einer ungeladenen Gruppe blockiert ist, die eine aliphatische Alkylgruppe mit mindestens 8 Kohlenstoffatomen enthält.

2. Insulinderivate nach Anspruch 1, dadurch gekennzeichnet, daß die B30-Carboxylgruppe mit einer ungeladenen Gruppe blockiert ist, die eine aliphatische Alkylgruppe mit 8 bis 12 Kohlenstoffatomen enthält.

3. Insulinderivate nach Anspruch 1, dadurch gekennzeichnet, daß die B30-Carboxylgruppe in Form eines Alkylamids mit mindestens 8 Kohlenstoffatomen blockiert ist.

4. Insulinderivate, bei denen die B30-Carboxylgruppe und eine bis vier der Carbonsäuregruppen an den Aminosäureresten der Positionen A4, A17, B13 und B21 des Insulinmoleküls mit ungeladenen Gruppen blockiert sind oder in ungeladene Gruppen umgewandelt sind, dadurch gekennzeichnet, daß die B30-Carboxylgruppe als Amid oder mit einer ungeladenen Gruppe, die eine aliphatische Alkylgruppe mit 1 bis 12 Kohlenstoffatomen enthält, blockiert ist, wobei Gln (A17), Gln (B13), Thr (B30)- $NH_2$-Humaninsulin, Gln (A17), Thr (B30)-$NH_2$-Humaninsulin und Gln (B13), Thr (B30)-$NH_2$-Humaninsulin ausgenommen sind.

5. Insulinderivate nach Anspruch 4, dadurch gekennzeichnet, daß die B30-Carboxylgruppe mit einer ungeladenen Gruppe blockiert ist, die eine aliphatische Alkylgruppe mit 8 bis 12 Kohlenstoffatomen enthält.

6. Insulinderivat nach Anspruch 4, dadurch gekennzeichnet, daß es ein Insulin-(A4, B30)-Diamid ist.

7. Insulinderivate nach einem der Ansprüche 1 bis 6, dadurch gekennzeichnet, daß sie Humaninsulinderivate sind.

8. Insulinderivate nach einem der Ansprüche 1 bis 6, dadurch gekennzeichnet, daß es Schweineinsulinderivate sind.

9. Insulinpräparationen, dadurch gekennzeichnet, daß sie mindestens ein Insulinderivat nach einem der Ansprüche 1 bis 8 enthalten.

10. Insulinpräparationen nach Anspruch 9, dadurch gekennzeichnet, daß sie ferner ein schnell wirkendes Insulin enthalten.

11. Insulinpräparationen nach Anspruch 9 oder 10, dadurch gekennzeichnet, daß sie Injektionspräparationen mit protrahierter Insulinwirkung darstellen und eine Suspension mindestens eines Insulinderivats in einem isotonischen wäßrigen Medium mit neutralem pH sowie wahlweise einen Puffer und/oder ein Konservierungsmittel enthalten.

**Revendications**

1. Dérivés d'insuline, dans lesquels le groupe carboxyle en B30 est bloqué par un groupe non chargé, caractérisés en ce que le groupe carboxyle en B30 de la molécule d'insuline est bloqué par un groupe non chargé comprenant un groupe alkyle aliphatique ayant au moins 8 atomes de carbone.

2. Dérivés d'insuline selon la revendication 1, caractérisés en ce que le groupe carboxyle en B30 est bloqué par un groupe non chargé comprenant un groupe alkyle aliphatique en $C_8$—$C_{12}$.

3. Dérivés d'insulin selon la revendication 1, caractérisés en ce que le groupe carboxyle est bloqué sous forme d'un alkylamide ayant au moins 8 atomes de carbone.

4. Dérivés d'insuline dans lesquels le groupe carboxyle en B30 et 1 à 4 des groupes acides carboxyliques sur les résidus d'aminoacides en positions (A4, A17, B13 et B21) et la molécule d'insuline sont bloqués par ou transformés en des groupes non chargés, caractérisés en ce que le groupe carboxyle en B30 est bloqué sous forme d'un amide ou par un groupe non chargé comprenant un groupe alkyle aliphatique en $C_1$—$C_{12}$, à l'exclusion de la Gln (A17), Gln (B13), Thr (B30)-$NH_2$-insuline humaine de la Gln (A17), Thr (B30)-$NH_2$-insuline humaine et de la Gln (B13), Thr (B30)-$NH_2$-insuline humaine.

5. Dérivés d'insuline selon la revendication 4, caractérisés en ce que le groupe carboxyle (B30) est bloqué par un groupe non chargé comprenant un groupe alkyle aliphatique en $C_8$—$C_{12}$.

6. Dérivés d'insuline selon la revendication 4, caractérisés en ce que c'est un insuline-(A4,B30)-diamide.

7. Dérivés d'insuline selon l'une quelconque des revendications 1 à 6, caractérisés en ce que ce sont des dérivés d'insuline humaine.

8. Dérivés d'insuline selon l'une quelconque des revendications 1 à 6, caractérisés en ce que ce sont des dérivés d'insuline de porc.

9. Préparations d'insuline caractérisées en ce qu'elles contiennent au moins un dérivé d'insuline selon l'une quelconque des revendications 1 à 8.

10. Préparations d'insuline selon la revendication 9, caractérisées en ce qu'elles contiennent également une insuline à action rapide.

11. Préparations selon la revendication 9 ou 10, caractérisées en ce que ce sont des préparations injectables présentant un effet d'insuline prolongé et qui comprennent une suspension d'au moins un dérivé d'insuline dans un milieu aqueux isotonique ayant un pH neutre et contiennent facultativement un tampon et/ou un agent conservateur.